# EUROPEAN PATENT SPECIFICATION

(11) **EP 2 185 673 B1**
(45) Date of publication and mention of the grant of the patent: **14.02.2018**
(21) Application number: 08787712.2
(22) Date of filing: 18.07.2008
(51) Int. Cl.: C11C 3/00, C07J 9/00

(54) **TRANSESTERIFICATION PROCESS**
UMESTERUNGSVERFAHREN
PROCÉDÉ DE TRANSESTÉRIFICATION

(30) Priority: 18.07.2007 FI 20070554
(43) Date of publication of application: 19.05.2010
(73) Proprietor: Raisio Nutrition Ltd., 21201 Raisio (FI)
(72) Inventor: EKBLOM, Jari, FI-21210 Raisio (FI); ORTE, Juha, FI-21280 Raisio (FI); HAMUNEN, Antti, FI-20100 Turku (FI)
(74) Representative: Berggren Oy, Helsinki & Oulu
(86) International application number: PCT/FI2008/050439
(87) International publication number: WO 2009/010641

(56) References cited:
- EP-A- 1 275 309
- WO-A-2004/089116
- US-A- 4 076 700
- US-A1- 2002 016 317
- US-B2- 6 713 466

## Description

### FIELD OF THE INVENTION

The invention relates to a new efficient process and device for applying transesterification.

### BACKGROUND OF THE INVENTION

The method conventionally used for preparing plant sterol fatty acid esters and/or plant stanol fatty acid esters is a batch type process consisting of blending the plant sterol and/or plant stanol and an alkyl ester of the fatty acid or fatty acid mixture, typically a methyl fatty acid ester, into a stirred tank reactor, drying the mixture by using elevated temperature and vacuum while agitating and adding a sufficient amount of a transesterification catalyst to the dried mixture. In the transesterification reaction plant sterol fatty acid ester and/or plant stanol fatty acid ester (often called only plant sterol ester and/or plant stanol ester) and methanol are produced. Methanol is removed as vapour from the reactor by vacuum and is condensed and collected at the vacuum system. The reaction is continued until a targeted reaction conversion of plant sterol ester and/or plant stanol ester is achieved.

One problem with the conventional plant sterol and/or plant stanol transesterification method is that after the transesterification catalyst has been added abound methanol formation occurs in the early stage of the reaction. Thus a large amount of methanol is vaporised through the bulk and vigorous foaming is thereby caused in the caustic reaction environment. Heavy foaming may cause product losses from the reactor headspace via the vacuum line (here called "over-foaming"). To avoid such losses the vigorous foaming has to be restricted. This can be done by reducing the reaction speed, which can be accomplished by decreasing the vacuum of the reactor system, by regulating agitation, by using lower temperature and/or by reducing the amount of catalyst. These changes all lead to longer reaction times and reduced production capacity. Also the control of the process is demanding i.e. a lot of manpower in manual operation or investments for process automation are required.

Foaming during the transesterification reaction reduces the production capacity also because the free headspace volume of the reactor has to be larger in order to avoid the reactor from over-foaming. This will require that only smaller operational batch sizes can be used since the reactor volume can be utilised only partly.

Furthermore, batch processing requires an elevated amount of transesterification catalyst in order to achieve a high enough esterification degree for fulfilling the requirement for commercial esterified plant sterols and/or esterified plant stanols, typically a degree of esterification of at least 95%. In the prior art the amount of catalyst used is from 0.2% to 1.2% by weight based on the plant sterol and/or plant stanol amount in the feed. Typically it is about 0.3% by weight of the plant sterol and/or plant stanol. (e.g. WO92/19640, WO98/06405 and WO99/56558).

Additionally, using a high amount of transesterification catalyst in the reaction makes the catalyst removal step more challenging. The higher amount of catalyst used, the more caustic conditions will be obtained when the catalyst is destroyed. Catalyst deactivation is done by adding water or using a dilute acidic solution containing a week acid such as citric acid, and more of them is needed if there is more catalyst to be destroyed. Furthermore, a higher amount of bleaching agent is needed. In combination all these are causing higher processing costs in the means of larger consumption of processing aids and increased waste treatment. Additionally, it is a known fact that catalyst consumption directly affects the product yield i.e. the more catalyst is used the more product will be lost to the waste stream due to saponification during work-up.

A batch process for producing plant sterol ester and/or plant stanol ester with a high degree of esterification requires typically a long reaction time in industrial production scale (about 2- 20 h depending of the batch size and process design). A long reaction time at elevated temperatures (about 100-130°C) and caustic environment will expose the feed components to degradation e.g. formation of free fatty acids by hydrolysis and isomerisation of fatty acids (e.g. conjugation of double bonds). Furthermore, the need to control the foaming leads to an elevated concentration of methanol in the reaction mixture, which results in an increased reformation of methyl fatty acid ester because the reaction is an equilibrium reaction.

The main problem in batch transesterification of plant sterols and/or plant stanols is therefore foaming. Static or rotating foam breakers or the use of a food grade defoamer has been shown to have only limited effect in avoiding foaming in this process application. A loop batch reactor with reflux-circulation through nozzles or distribution disks have some effect in destroying the foam, but do not prevent over-foaming of the reactor if high vacuum at a reaction temperature of 100-130°C is used already in the beginning of the reaction.

Batch and continuous interesterification of edible oils and fats is disclosed in J. Am. Oil Chemists' Soc., 55, 796-805 (1978), Sreenivasan, B., Interesterification of Fats. Here the continuous interesterification method consists of heating the oil to desired processing temperature, drying the oil by vacuum in an oil dryer to bring the moisture level down to about 0.01%. Sodium metal is introduced into the dried hot oil as catalyst. The mixture of catalyst in oil is homogenised to disperse the catalyst and the mixture is then passed through a tubular reactor. Residence time is varied by changing the reactor coil length. The catalyst is deactivated by adding water in a mixer, and soap and oil are separated by centrifugation. The washed oil is dried in an oil dryer and pumped to a storage tank. However, this process is not suitable for plant sterol fatty acid ester and/or plant stanol fatty acid ester manufacturing because there is no means for removal of the volatile methanol produced.

US Patent 6713466 teaches that plant sterol ester can be manufactured by using calcium oxide, calcium hydroxide, a calcium salt of a carboxylic acid, or magnesium hydroxide as catalyst in a direct esterification process of sterols using free fatty acids. The disclosure deals with conventional batch processing and there is mentioned the possibility of using a combination of a stirred tank and a thin film evaporator, or a thin film evaporator with one or more runs through the evaporator to remove the formed water. The processing method described has the disadvantage that the needed reaction temperature is high, e.g. 190-230°C in order to achieve a sufficient reaction conversion. As also mentioned in the document, there is already a serious risk for sterol dehydration when such high temperature has to be used. The high temperature exposes the feed also to *trans* fatty acid formation which will decrease the nutritional value of the product and even render it impossible to use in market areas where so-called functional foods have to be free from *trans* fatty acids. Furthermore, the degree of esterification of the product produced by the method described will be low because the catalysts used have too low activity. Therefore, if using lower reaction temperatures e.g. 100-130°C practically no reaction will occur. Also the reaction time is rather long e.g. in examples 1- 4 disclosing laboratory scale experiments is mentioned 6-12 hours. The processing costs of the plant sterol ester produced by this esterification method becomes uneconomically high because of the high energy costs due to the long processing time and high temperature needed. The esterification reaction taught by US Patent 6713466 if applied in industrial scale would lead to even longer reaction and processing times as compared to the laboratory scale experiments. Thus, from a practical point of view applying this esterification reaction would lead to an industrial scale operation with poor economy. Furthermore, the obtained plant sterol ester would be of nutritionally unacceptable quality.

Therefore, an improved process is needed to avoid problems mentioned with the above disclosed esterification methods.

### SUMMARY OF THE INVENTION

The invention is based on the surprising finding that feeding continuously a reaction mixture comprising sterols and/or stanols, a fatty acid or fatty acid mixture in the form of an alkyl ester, typically methyl fatty acid ester, and a transesterification catalyst through a thin film evaporator results in an efficient transesterification reaction. This continuous manufacturing method leads to higher production throughput and improves the overall processing economics by reducing the catalyst consumption and increasing the product yield and simultaneously improving the product quality due to the shortened reaction time.

The present invention relates to a process for the preparation of sterol and/or stanol esters by using a continuous transesterification process. For a start a sterol and/or stanol and an alkyl fatty acid ester are dried at elevated temperature and reduced pressure. This is preferably performed continuously. The transesterification reaction is then carried out by pumping the dried feed continuously and adding and preferably mixing a transesterification catalyst continuously in-line, transferring continuously the obtained reaction mixture of reactants and catalyst through a thin film evaporator operating at elevated temperature and low pressure. The transesterification reaction results in a fast and even an almost complete transesterification by effectively removing alkanol. The obtained reaction product, mainly esterified plant sterols and/or stanols (degree of esterification e.g. at least 90%, preferably at least 95% and more preferably at least 98%) is treated to remove the catalyst and is finally refined e.g. by deodorising. Also, catalyst removal and refining, including optional bleaching and deodorising, is preferably performed continuously.

The transesterification reaction can be initiated in a pre-reactor unit wherefrom the formed volatile alkanol is removed. It is also possible to continuously recirculate part of the sterol/stanol fatty acid ester product recovered from the thin film evaporator into the thin film evaporator or into the pre-reactor unit. By means of the pre-reactor unit and/or recirculation less alkanol is formed in the thin film evaporator and therefore the possible foaming and splashing problem in the thin film evaporator is decreased or even eliminated.

The present invention also relates to a device for the continuous preparation of a sterol and/or stanol fatty acid ester.

### DETAILED DESCRIPTION OF THE INVENTION

According to a first aspect of the present invention there is provided a process for the preparation of a sterol and/or stanol fatty acid ester by a continuous transesterification process, said preparation process comprises providing a dried feed mixture comprising a sterol and/or stanol and an alkyl fatty acid ester, adding a transesterification catalyst to the dried feed mixture to obtain a reaction mixture, continuously feeding the reaction mixture into a thin film evaporator where the transesterification reaction takes place, continuously withdrawing alkanol formed during the reaction from the thin film evaporator, and continuously recovering a sterol and/or stanol fatty acid ester product from the thin film evaporator, and removing catalyst from the recovered product, and optionally refining the recovered product, wherein said reaction mixture entering the thin film evaporator comprises a partly transesterified reaction mixture.
The addition of the transesterification catalyst is made more efficient by mixing the transesterification catalyst in-line with the dried feed mixture.

This partly transesterified reaction mixture entering the thin film evaporator can be obtained by continuously feeding the reaction mixture into a pre-reactor unit where the transesterification reaction is initiated and/or maintained and alkanol formed during the reaction is removed.
The pre-reactor unit can comprise a vapour-liquid separator such as a flash tank, flash separator, degasser, cyclone separator, packed or tray column or a reactor equipped with an agitator. Preferably the pre-reactor unit is a flash tank, flash separator, degasser, cyclone separator, packed or tray column, more preferably it is a flash tank, flash separator, degasser or cyclone separator.

The pressure of the reaction mixture in between the catalyst addition and the pre-reactor unit is preferably in the range from 10 to 1000 kPa, more preferably from 50 to 500 kPa and, the temperature is preferably in the range from 100 to 170°C, more preferably from 120 to 140 °C. The residence time of the reaction mixture in between the catalyst addition and pre-reactor unit is preferably from 0.1 to 180 seconds, more preferably from 0.2 to 60 seconds, and most preferably from 0.5 to 30 seconds.

In case a pre-reactor unit is not present, the pressure of the reaction mixture in between the catalyst addition and the thin film evaporator is preferably in the range from 10 to 1000 kPa, more preferably from 50 to 500 kPa, and the temperature is preferably in the range from 100 to 170°C, more preferably from 120 to 140 °C. The residence time of the reaction mixture in between the catalyst addition and thin film evaporator is preferably from 0.1 to 180 seconds, more preferably from 0.2 to 60 seconds, and most preferably from 0.5 to 30 seconds.

The partly transesterified reaction mixture entering the thin film evaporator can also be obtained by combining the reaction mixture with a continuously recirculated part of the sterol and/or stanol fatty acid ester product recovered from the thin film evaporator, and/or by combining the dried feed mixture with a continuously recirculated part of the sterol and/or stanol fatty acid ester product recovered from the thin film evaporator and adding the catalyst to the mixture of dried feed mixture and recirculated part.

According to the present invention it is also possible to have both a pre-reactor unit and the above recirculation. In that case the continuously recirculated part of the sterol and/or stanol fatty acid ester product can be combined with the reaction mixture before and/or after the pre-reactor unit, and/or it can be combined with the dried feed mixture, whereafter the catalyst is added to form the partly transesterified reaction mixture before the pre-reactor unit. This expression including three "and/or" means the process according to the invention may include one, two or three of the recirculation streams mentioned; before the pre-reactor unit, after the pre-reactor unit and to the dried feed mixture.

In case a pre-reactor unit is present, the weight ratio of the recirculated part of the sterol and/or stanol fatty acid ester product to native reaction mixture is preferably from 0.01:1 to 10:1, more preferably from 0.05:1 to 5:1, even more preferably from 0.1:1 to 3:1 and most preferably from 0.1:1 to 0.5:1 (weight/weight).
In case a pre-reactor unit is not present, the weight ratio of the recirculated part of the sterol and/or stanol fatty acid ester product to native reaction mixture is preferably from 0.1:1 to 10:1, more preferably from 1:1 to 5:1, and most preferably from 1:1 to 3:1 (weight/weight).
In a second aspect of the present invention there is provided a device for the continuous preparation of a sterol and/or stanol fatty acid ester by a continuous transesterification process, said device comprising
means for providing a continuous stream of dried feed mixture comprising a sterol and/or stanol and an alkyl fatty acid ester;
means for continuous introduction of a transesterification catalyst into the dried feed mixture to obtain a reaction mixture;
means for continuous feeding of the reaction mixture into a thin film evaporator, said thin film evaporator being equipped with inlet means for introduction of the reaction mixture, first outlet means for continuous withdrawal of a sterol and/or stanol fatty acid ester product from the thin film evaporator and second outlet means for continuous removal of volatile components; and
means for regulation of temperature and pressure,
said device additionally comprising a pre-reactor unit being positioned in the flow direction before the thin film evaporator, wherein the pre-reactor unit is a reaction vessel including means for removal of vapours, and wherein the pre-reactor unit contains no mechanical agitation and is a flash tank, flash separator, degasser, cyclone separator, packed or tray column.
The device according to the invention may additionally include an in-line mixer unit for mixing the introduced transesterification catalyst with the dried feed mixture.

Preferably the pre-reactor unit is a flash tank, flash separator, degasser or cyclone separator.
This device including the pre-reactor unit may additionally comprise means for continuous recirculation of part of the sterol and/or stanol fatty acid ester product withdrawn from the thin film evaporator into any of the following: the reaction mixture entering the pre-reactor unit (5), the partly esterified reaction mixture entering the thin film evaporator (7) and the dried feed mixture, or any combination thereof.

According to another embodiment not according to the invention where the device does not include a pre-reactor unit, the device additionally comprises means for continuous recirculation of part of the sterol and/or stanol fatty acid ester product withdrawn from the thin film evaporator into the reaction mixture entering the thin film evaporator and/or into the dried feed mixture.
The device of the invention may additionally comprise a catalyst removal unit and a refining unit.
The device according to the invention may additionally comprise a unit for continuous drying of feed mixture to provide said continuous stream of dried feed mixture.
The term "sterol and/or stanol" includes all desmethyl, 4-monomethyl and 4,4'-dimethyl sterols as well as corresponding hydrogenated stanol forms. The term also includes sterol-like compounds i.e. triterpene alcohols. The most important sterols are the desmethyl sterols, especially sitosterol, campesterol and stigmasterol, and corresponding stanols sitostanol and campestanol. In this specification the sterols and/or stanols include plant sterols and/or plant stanols as well as cholesterol and/or cholestanol.
The transesterification process according to the present invention uses as fatty acid source alkyl fatty acid esters, often called only alkyl esters, such as lower alkyl fatty acid esters having an alkyl length of C1-4, which include methyl, ethyl, propyl or butyl fatty acid esters, or higher alkyl fatty acid esters (C5-10) such as pentyl, hexyl, heptyl fatty acid esters. Preferably the alkyl fatty acid esters to be used are lower alkyl C1-4 fatty acid esters, more preferably methyl or ethyl fatty acid esters, and most preferably methyl fatty acid esters. In the transesterification reaction a second reaction product alkanol, preferably lower alkanol C1-4, e.g. methanol is formed from the alkyl fatty acid esters besides the first reaction product, the sterol and/or stanol fatty acid ester.

By "fatty acid" is in this specification meant any carboxylic acid with a length of C2-24, preferably a length of C12-20. The fatty acids include saturated, monoun-saturated and/or polyunsaturated fatty acids. The fatty acid can be an individual fatty acid or a mixture of two or several fatty acids. Preferably they are obtained from triglyceride oils and/or fats such as edible vegetable oils and/or fats or edible marine oils and/or fats. Preferably the fatty acid can have the fatty acid composition of a triglyceride oil or fat or a mixture of triglyceride oils and/or fats or it can be a certain fraction of the fatty acids of a triglyceride oil and/or fat. Preferably the fatty acids contain a high amount of polyunsaturated fatty acid, preferably at least 25%, more preferably at least 35% and most preferably at least 45% of polyunsaturated fatty acids of the total fatty acids. In the transesterification reaction the fatty acid-residue is transferred from the alkyl fatty acid ester to the sterol and/or stanol to form sterol and/or stanol fatty acid ester while the formed alkanol is efficiently removed.

As catalyst in the transesterification reaction according to the invention alkali metals such as sodium or alkali metal alcoholates are used. Preferably the catalyst is an alkali metal alcoholate and preferably the alkali metal alcoholate is sodium methylate or sodium ethylate. Also potassium alcoholates can be used. The amount of transesterification catalyst is typically from 0.01 to 2% by weight, more typically from 0.05 to 1% and most typically from 0.1 to 0.5% of the weight of the sterol and/or stanol used in the transesterification. The amount of catalyst is typically less than needed in corresponding batch processing e.g. from 0.01 to less than 0.2% by weight, typically from 0.02 to 0.19% by weight, more typically from 0.05 to 0.18% by weight, still more typically from 0.05 to 0.17% by weight, and most typically from 0.05 to 0.16% by weight of the sterol and/or stanol.

The catalyst mixer unit is preferably an in-line static mixer or a motor driven mixer such as a knife mixer or a paddle mixer. By using a catalyst mixer unit the catalyst will be thoroughly distributed into the dried feed. Preferably the catalyst is mixed by using a static mixer.

By degree of esterification is here meant the molar % of sterol and/or stanol fatty acid ester to the total sterol and/or stanol of the product after the pre-reactor unit or of the product recovered from the thin film evaporator.

The pre-reactor unit according to the invention is a reaction vessel including means for removal of vapours. Known commercial vessels suitable for this are for example vapour-liquid separators, such as flash tanks, flash separators, degassers, or centrifugal vapour-liquid separators such as cyclone separators. These are originally meant for removing volatile compounds by decreased pressure and/or increased temperature from a liquid. This pre-reactor unit is very simple in construction and contains preferably no movable parts, thus being an economical investment, having low operation costs and needs for maintenance. Packed or tray columns may also be used as pre-reactors. The pre-reactor contains no mechanical agitation. By having no mechanical agitation foaming is reduced. Furthermore, it is obvious that a thin film evaporator is not included in the definition of a pre-reactor according to the invention.
The shape of the pre-reactor according to the invention is typically vertical, horizontal or spherical. The pre-reactor is equipped with reaction mixture inlet, vapour outlet and esterified reaction mixture outlet pipelines. In order to achieve uniform feed rate and superior vapour-liquid separation, and additionally to avoid foam formation, the reaction mixture is typically transferred into the upper section of the pre-reactor above the liquid level by using a feed distributor. Any type of feed distributor may be used, such as a vane or a vapour horn distributor, a ring or disk distributor, an orifice pan or an orifice trough distributor, a bare or a rounded V-baffle nozzle, a ladder pipe or a spray distributor. In the case of a cyclone type of pre-reactor the reaction mixture inlet is typically mounted tangentially. A mist extractor, also commonly called a demister or a mist eliminator, is preferably installed inside the pre-reactor, typically in between the feed inlet section and the vapour outlet, to remove liquid reaction mixture or/and esterified reaction mixture droplets entrained in the flowing vapour stream. The outlet of the esterified reaction mixture is typically installed on the bottom of the pre-reactor. Optionally, the pre-reactor according to the invention may include structured or random packings, vortex breakers, baffles and/or measurement devices such as temperature, pressure or level gauges. To avoid flashing of volatile compounds included in the reaction mixture prior to the pre-reactor, the reaction mixture line is equipped with a back-pressure valve, which is optimally located close to the pre-reactor. A continuously operating reactor equipped with an agitator can also be used as pre-reactor, but it is more expensive to buy and maintain due to more complex construction. In the mentioned pre-reactor types the residence time of the reaction mixture can be adjusted by regulating the amount of reaction mixture fed into the pre-reactor unit and/or by the vessel volume.

Preferably the pre-reactor unit according to the invention is operated under vacuum and is preferably equipped with a heating system to compensate the heat removal due to the distillation of the volatile alkanol. An inert gas such as nitrogen may optionally be added into the reaction mixture to help the removal of volatile alkanol. The size of the pre-reactor unit has to be designed based on the targeted production capacity so that the flashed alkanol vapour can be evacuated without significant pressure increase in the system.
Preferably the operating pressure in the pre-reactor unit should be at most 95 kPa, more preferably at most 10 kPa and most preferably at most 3 kPa. Preferably the operating temperature in the pre-reactor unit is in the range of from 90 to 200°C, more preferably from 100 to 170°C and most preferably from 110 to 150°C. A typical residence time of the reaction mixture in the pre-reactor unit is from 0.1 to 300 seconds, more typically from 0.2 to 180 seconds and most typically from 0.5 to 60 seconds. The degree of esterification achieved in the pre-reactor unit according to the invention typically varies from 5 to 94%, more typically from 10 to 90%, even more typically from 15 to 85%, still more typically from 20 to 80% and most typically from 30 to 70%.
The thin film evaporator according to the invention may be a falling film evaporator, a wiped film evaporator, a short path evaporator or any other possible evaporator forming thin films.

In case no pre-reactor unit is present, in an embodiment not according to the invention, to avoid flashing of the volatile compounds included in the reaction mixture prior to the thin film evaporator, the reaction mixture line is typically equipped with a back-pressure valve, which is optimally located close to the thin film evaporator.
Inert gas such as nitrogen may optionally be added into the reaction mixture to help volatile alkanol removal in the thin film evaporator.
Preferably the operating pressure in the thin film evaporator should be at most 95 kPa, more preferably at most 10 kPa and most preferably at most 3 kPa. Preferably the operating temperature in the thin film evaporator is in the range of from 90 to 200°C, more preferably from 100 to 170°C and most preferably from 110 to 150°C. A typical residence time of the reaction mixture in the thin film evaporator is from 0.1 to 180 seconds, more typically from 0.2 to 60 seconds and most typically from 0.5 to 30 seconds.

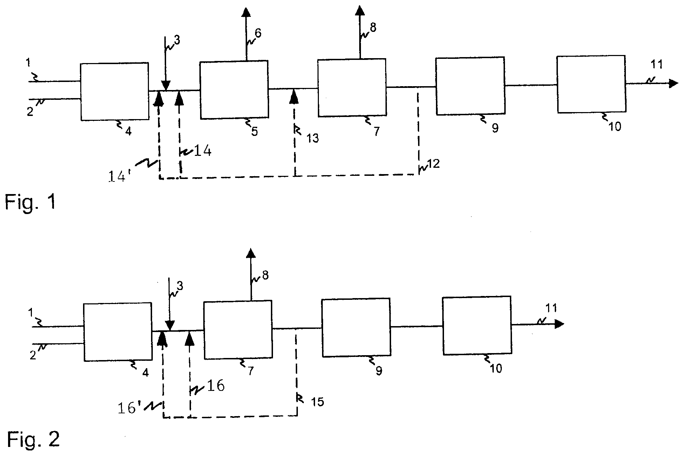

The molar ratio of the sterol and/or stanol reactant to the alkyl fatty acid reactant is preferably in the range from 1:0.95 to 1:3, more preferably from 1:0.97 to 1:2, and most preferably from 1:0.99 to 1:1.5.
The degree of esterification achieved using a pre-reactor unit in combination with a thin film evaporator typically varies from 75 to 100%, more typically the degree of esterification is at least 85%, still more typically at least 95%. A degree of esterification of at least 98% can quite easily be obtained.
The pre-reactor unit and the thin film evaporator according to the invention can be made of separate equipments or optionally these are combined in one.
The vacuum system of the transesterification process according to the invention contains typically piping from the pre-reactor unit, if used, and from the thin film evaporator for removing the second reaction product, the alkanol, a condenser for the alkanol, and conventional vacuum equipment. Any vacuum equipment may be used, such as a water-ring pump, a rotary vane pump, or vacuum ejectors or any combinations thereof. The vacuum system can be shared with both the pre-reactor unit and the thin film evaporator, or both equipments can have separate vacuum systems. The size and capacity of the vacuum system has to be designed based on the targeted production capacity and the operation pressure so that the flashed alkanol vapour can be evacuated without significant pressure increase in the system. Formed alkanol is collected from the pre-reactor unit and from the thin film evaporator and is condensed prior to or after the vacuum equipment.
The continuous transesterification process according to the invention and the device for carrying out the process are described below in more detail with reference to the enclosed drawing wherein
- Fig.1: shows schematically a device according to an embodiment of the invention, and
- Fig.2: shows schematically a device according to another embodiment not according to the invention.
Referring to Fig. 1 the sterol and/or stanol reactant 1 and the alkyl fatty acid ester reactant 2 (preferably fatty acid methyl ester) are mixed by using a ratio from 1:0.95 to 1:3, more typically from 1:0.97 to 1:2 and most typically from 1:0.99 to 1:1,5 (mol/mol) of sterol and/or stanol to alkyl fatty acid ester. Preferably this ratio is fixed in a steady working process. This mixture is here called feed. The feed is then dried by using agitation, vacuum and a temperature of e.g. 100-130°C to remove the moisture optimally to a level of less than 0.01% by weight. Preferably mixing of the feed components is performed continuously in-line, typically within the fixed ratio, the feed is heated to totally dissolve sterols and/or stanols and drying of the dissolved feed is performed continuously e.g. in a dryer unit 4 (temperature preferably 100-150°C, more preferably 100-130°C and pressure preferably less than 5 kPa, most preferably less than 3 kPa). The feed is pumped or sprayed into the dryer, the volatile compounds such as residual water are evaporated and the resulting dried feed is pumped out from the bottom of the dryer 4.

The transesterification catalyst 3 is added continuously in-line to the dried feed stream preferably by using a proportional dosing ratio of catalyst to sterol and/or stanol. Preferably the catalyst is further mixed in a mixer unit e.g. by using an in-line static mixer or a motor driven mixer such as a knife mixer or a paddle mixer (not shown in the figures) to distribute the catalyst thoroughly in the dried feed. Preferably the catalyst is mixed by using a static mixer. If a catalyst in powder form is used, it is preferably first dispersed or dissolved in a suitable solvent such as lower alkanol or alkyl fatty acid ester to enable accurate dosing of the catalyst by e.g. a metering pump.

After mixing the catalyst with the dried feed the resulting reaction mixture is pumped further via the process line. The process line in between the catalyst mixer and the pre-reactor 5 is dimensioned so that it allows the transesterification reaction to start. A typical residence time of the reaction mixture in between the catalyst addition and pre-reactor (i.e. the inlet of the pre-reactor) is from 0.1 to 180 seconds, more typically from 0.2 to 60 seconds and most typically from 0.5 to 30 seconds. A typical pressure of the reaction mixture in between the catalyst addition and the pre-reactor is from 10 to 1000 kPa, preferably from 50 to 500 kPa and the temperature is from 100 to 170°C, preferably from 120 to 140 °C.

In this improved processing method utilising both a pre-reactor unit 5 and a thin film evaporator 7 the reaction mixture is transferred into a pre-reactor unit which functions as a reaction vessel which also evaporates the formed volatile alkanol. The alkanol is transferred via the pre-reactor vapour outlet 6 and collected through the vacuum system after condensation. The reaction rate of the sterol and/or stanol ester in the pre-reactor unit can be controlled by changing the feed rate, temperature and vacuum level. Typical vacuum in the pre-reactor is from 0.1 to 10 kPa, preferably from 0.5 to 2 kPa and temperature from 100 to 170°C, preferably from 120 to 140°C. A typical residence time of the reaction mixture in the pre-reactor is from 0.1 to 300 seconds, more typically from 0.2 to 180 seconds and most typically from 0.5 to 60 seconds.

After the pre-reactor unit 5 the partly esterified reaction mixture is preheated if needed to the operating temperature of the thin film evaporator 7 by using an in-line heat exchanger. The reaction mixture is continuously transferred from the pre-reactor 5 to a thin film evaporator 7 which functions as a reactor which also evaporates the formed second reaction product 8 i.e. methanol in case methyl fatty acid ester is used. The reaction rate of the sterol and/or stanol ester in the thin film evaporator can be controlled by changing the feed rate, temperature and vacuum level. Typical operation vacuum in the evaporator is from 0.1 to 10 kPa, preferably from 0.1 to 2 kPa and temperature from 100 to 170°C, preferably from 120 to 140°C. Sterol and/or stanol ester is pumped out as a first reaction product and alkanol as a second reaction product 8 is collected through the vacuum system of the evaporator after condensing. The alkanol can be collected separately from the pre-reactor unit and from the thin film evaporator, but preferably the condensation is performed in the same equipment. A typical residence time of the reaction mixture in the thin film evaporator is from 0.1 to 180 seconds, more typically from 0.2 to 60 seconds and most typically from 0.5 to 30 seconds. The reaction is completed to a degree of esterification of preferably at least 90%, more preferably at least 95%, and most preferably at least 98%.

When the combination process including a pre-reactor unit 5 and a thin film evaporator 7 according to the invention is used, 1 m² surface area of the thin film evaporator heating cylinder leads typically to a production capacity of from 1 to 1000 kg/h, more typically from 5 to 500 kg/h and most typically from 10 to 300 kg/h of sterol and/or stanol ester based on the sterol and/or stanol amount used in the feed. The achieved capacity is increased, typically at least doubled and preferably at least tripled, compared to a process model in which the pre-reactor is excluded and only a thin film evaporator is used for reaction purposes. The shear of the total transesterification reaction of the present invention that is performed in the pre-reactor unit is typically from 5 to 94%, more typically from 20 to 80% and most typically from 30 to 70%.

After the reaction the catalyst is removed in unit 9 by water washing or neutralisation with acid and the material is optionally bleached and further deodorised in unit 10 to achieve proper quality of the sterol and/or stanol ester 11 to be used for various food applications or other applications. The catalyst removal, the optional bleaching and the deodorisation can be performed batch-wise, but are preferably continuously working process steps.

The broken lines 12, 13, 14 and 14' in Fig. 1 indicate that the recirculation of the reaction product is optional.

Typically there is no need for recirculating the reaction product through the thin film evaporator 7 if the reaction mixture feed rate is limited to a suitable level so that the process conditions are at steady state and the reaction product fulfills the reaction conversion specification. A too high feed rate of reaction mixture will cause unstable process conditions resulting in splashing leading to reaction product losses as well as reduced degrees of esterification. However, according to the invention it is possible to recirculate continuously a certain part of the recovered reaction product containing partly esterified sterol and/or stanol from the thin film evaporator 7 and mix this part prior to the thin film evaporator 7 with native reaction mixture thus preferably having a fixed ratio of reaction product to native reaction mixture. It is also possible to recirculate continuously a certain part of the recovered reaction product containing partly esterified sterol and/or stanol from the thin film evaporator 7 and introduce this part to the dried feed mixture prior to the catalyst addition or prior to the catalyst mixer unit. More preferably though the re-circulated part is introduced to the native reaction mixture, i.e. prior to the pre-reactor unit, and most preferably prior to the thin film evaporator. Referring to Fig. 1 the recirculated part 12 of the recovered reaction product can be introduced as stream 14 prior to the pre-reactor 5, as stream 13 between the pre-reactor 5 and the thin film evaporator 7, as stream 14' to the dried feed mixture, or as any combination thereof. A typical mixing ratio of reaction product to native reaction mixture is from 0.01:1 to 10:1, more typically from 0.05:1 to 5:1, even more typically from 0.1:1 to 3:1 and most typically from 0.1:1 to 0.5:1 by weight. The benefit of recirculating is to stabilise the operation of the thin film evaporator, because a relatively smaller portion of alkanol is released and splashing of the reactants can thereby be limited.

As explained above there is typically no need for product recirculation in a process including both a pre-reactor unit 5 and a thin film evaporator 7 since enough high reaction conversion is achievable by one run. The one-run mode maximises the utilisation of the production units resulting in high throughputs. However, it is possible according to the invention to recirculate continuously part 12 of the recovered reaction product containing partly esterified sterol and/or stanol from the thin film evaporator 7 back into the thin film evaporator 7 or even back to the pre-reactor 5 to improve the reaction conversion.

According to the present invention it has surprisingly been discovered that the pre-reactor concept according to the invention effectively prevents the foaming of the reaction mixture and increases the removal of the volatile second reaction product i.e. alkanol such as methanol. Thus, as a result a considerable part of the reaction (most typically 30-70%) can be performed already in the pre-reactor resulting in higher production capacity of the thin film evaporator.

Referring to Fig. 2 the sterol and/or stanol reactant 1 and the alkyl fatty acid ester reactant 2 (preferably fatty acid methyl ester) are mixed by using a ratio from 1:0.95 to 1:3, more typically from 1:0.97 to 1:2 and most typically from 1:0.99 to 1:1,5 (mol/mol) of sterol and/or stanol to alkyl fatty acid ester. Preferably this ratio is fixed in a steady working process. This mixture is here called feed. The feed is then dried by using agitation, vacuum and a temperature of e.g. 100-130°C to remove the moisture optimally to a level of less than 0.01% by weight. Preferably mixing of the feed components is performed continuously in-line typically within the fixed ratio, the feed is heated to totally dissolve sterols and/or stanols and drying of the dissolved feed is performed continuously e.g. in a dryer unit 4 (temperature preferably 100-150°C, more preferably 100-130°C and pressure preferably less than 5 kPa, most preferably less than 3 kPa). The feed is pumped or sprayed into the dryer, the volatile compounds such as residual water are evaporated and the resulting dried feed is pumped out from the bottom of the dryer 4.

The transesterification catalyst 3 is added continuously in-line to the dried feed stream preferably by using a proportional dosing ratio of catalyst to sterol and/or stanol. Preferably the catalyst is further mixed in a mixer unit e.g. by using an in-line static mixer or a motor driven mixer such as a knife mixer or a paddle mixer (not shown in the figures) to distribute the catalyst thoroughly into the dried feed. Preferably the catalyst is mixed by using a static mixer. If a catalyst in powder form is used, it is preferably first dispersed or dissolved in a suitable solvent such as lower alkanol or alkyl fatty acid ester to enable accurate dosing of the catalyst by e.g. a metering pump.

After mixing the catalyst with the dried feed the resulting reaction mixture is pumped further via the process line. The process line in between the catalyst mixer unit and the thin film evaporator 7 is dimensioned so that it allows the transesterification reaction to start. A typical residence time of the reaction mixture in between the catalyst addition and thin film evaporator (i.e. the inlet of the evaporator) is from 0.1 to 180 seconds, more typically from 0.2 to 60 seconds and most typically from 0.5 to 30 seconds. A typical pressure of the reaction mixture in between the catalyst addition and the thin film evaporator is from 10 to 1000 kPa, preferably from 50 to 500 kPa and the temperature is from 100 to 170°C, preferably from 120 to 140 °C.

The reaction mixture is continuously transferred from the catalyst mixer to the thin film evaporator 7 which functions as a reactor which also evaporates the formed second reaction product 8 i.e. methanol in case methyl fatty acid ester is used. The reaction rate of the sterol and/or stanol ester in the thin film evaporator can be controlled by changing the feed rate, temperature and vacuum level. Typical operation vacuum in the evaporator is from 0.1 to 10 kPa, preferably from 0.1 to 2 kPa and temperature from 100 to 170°C, preferably from 120 to 140°C. Sterol and/or stanol ester is pumped out as a first reaction product and alkanol as a second reaction product 8 is collected through the vacuum system of the evaporator after condensing. A typical residence time of the reaction mixture in the thin film evaporator is from 0.1 to 180 seconds, more typically from 0.2 to 60 seconds and most typically from 0.5 to 30 seconds. The reaction is completed to a degree of esterification of preferably at least 90%, more preferably at least 95%, and most preferably at least 98%.

When the process contains only a thin film evaporator 7 as reactor (without a pre-reactor unit) according to the invention, 1 m² surface area of the thin film evaporator heating cylinder leads typically to a production capacity of from 0.2 to 100 kg/h, more typically from 0.5 to 50 kg/h and most typically from 1 to 30 kg/h of sterol and/or stanol ester based on the sterol and/or stanol amount used in the feed.

After the reaction the catalyst is removed in unit 9 by water washing or neutralisation with acid and the material is optionally bleached and further deodorised in unit 10 to achieve proper quality of the sterol and/or stanol ester 11 to be used for various food applications or other applications. The catalyst removal, the optional bleaching and the deodorisation can be performed batch-wise, but are preferably continuously working process steps.

The broken lines 15, 16 and 16' in Fig. 2 indicate that the recirculation of the reaction product is optional.

Typically there is no need for recirculating the reaction product through the thin film evaporator 7 if the reaction mixture feed rate is limited to a suitable level so that the process conditions are at steady state and the reaction product fulfills the reaction conversion specification. A too high feed rate of reaction mixture will cause unstable process conditions resulting in splashing leading to reaction product losses as well as reduced degrees of esterification. However, according to the invention it is possible to recirculate continuously a certain part of the recovered reaction product containing partly esterified sterol and/or stanol from the thin film evaporator 7 and mix this part prior to the thin film evaporator 7 with native reaction mixture thus preferably having a fixed ratio of reaction product to native reaction mixture. It is also possible to recirculate continuously a certain part of the recovered reaction product containing partly esterified sterol and/or stanol from the thin film evaporator 7 and introduce this part to the dried feed mixture prior to the catalyst addition or prior to the catalyst mixer unit. More preferably though the re-circulated part is introduced to the native reaction mixture i.e. prior to the thin film evaporator. Referring to Fig. 2 the recirculated part 15 of the recovered reaction product is introduced prior to the thin film evaporator 7 as stream 16, to the dried feed mixture as steam 16', or the recirculated part is introduced as a combination of both streams. A typical mixing ratio of reaction product to native reaction mixture is from 0.1:1 to 10:1, more typically from 1:1 to 5:1, and most typically from 1:1 to 3:1. The benefit of recirculating is to stabilise the operation of the thin film evaporator, because a relatively smaller portion of alkanol is released and splashing of the reactants can thereby be limited.

In the embodiment described above with reference to Fig. 2, the recirculation is more beneficial than in the embodiment described above with reference to Fig. 1.

The processing time for the transesterification method according to the invention is shortened dramatically compared to a batch processing mode of several hours. This is because the foaming can be practically eliminated, especially when a combination process of a pre-reactor unit and a thin film evaporator is used. The reaction time i.e. the time in between catalyst is added and the sterol and/or stanol ester leaves the thin film evaporator is short, typically less than 20 minutes, preferably less than 10 minutes, more preferably less than 5 minutes and most preferably less than 2 minutes. Thus superior product quality can be achieved because heat induced degradation of both sterols and/or stanols and of fatty acids can be limited or practically eliminated due to the short residence time at elevated temperatures and alkaline conditions.

Processing costs for the transesterification method according to the invention can be lowered significantly for several reasons. Firstly, less catalyst is needed in order to produce sterol and/or stanol esters with a high degree of esterification. Further, smaller catalyst consumption leads to a higher product mass yield and lower waste costs, as well as easier catalyst removal. Also operational costs of the proposed machinery are significantly lower compared to traditional batch-type machinery, which boosts the overall productivity of a plant. Additionally, investment costs are lower, because the sizes of the vessels are small relative to batch vessels of the same capacity.

### Reference Example 1

### A conventional batch process

50.0 kg of rapeseed oil methyl ester (a methyl fatty acid ester with fatty acids from low erucic acid rapeseed oil) and 50.0 kg of plant stanols (molar ratio 1.4:1) were loaded into a pilot scale reactor and this mixture was dried to remove water at 120°C and 1 kPa for 30 minutes with agitation. After drying the vacuum was broken with nitrogen and 0.35 wt-% of sodium methylate catalyst based on the plant stanol amount was added as a 30 wt-% solution in methanol. Reaction temperature was set to 125°C and pressure was slowly reduced from atmospheric in order to control intensive foaming due to the methanol removal from the reaction mixture. Methanol formed during the reaction was vaporised and transferred from the reactor headspace by vacuum and condensed at the vacuum system. Samples were taken during the reaction after 15, 30, 60 and 120 minutes. During final sampling at 120 minutes the vacuum was 1.7 kPa, whereupon the reaction was stopped by breaking the vacuum with nitrogen. The degree of esterification of each sample was calculated from the GC analysis of the stanol fatty acid ester and the free stanol. The degrees of esterification were 76.2% after 15 min, 88.1% after 30 min, 93.4% after 60 min, and 95.4% after 120 min.

The test was repeated by using only 0.17 wt-% of catalyst in the reaction. The degrees of esterification were then 55.0% after 15 min, 74.3% after 30 min, 86.6% after 60 min, and 89.4% after 120 min.

The results show unacceptable degrees of esterification when less catalyst is used. Also heavy foaming and splashing had to be controlled by regulating the vacuum level in both batch reactions thus reducing the reaction speed dramatically resulting in a long processing time. Additionally, due to the splashing some reaction product entered the vacuum line causing loss and a lower yield.

### Example 2

### The use of a thin film evaporator as reactor

50.0 kg of rapeseed oil methyl ester and 50.0 kg of plant stanols (molar ratio 1.4:1) were loaded into a feed vessel and this mixture was dried to remove water at 120°C and 1 kPa for 30 minutes with agitation. After drying the feed was pumped continuously from the feed tank with a feed rate of 61 kg/h to the reactor system consisting of a thin film evaporator (short path evaporator) with a heating area of 0.45 m². 0.17 wt-% of sodium methylate catalyst based on the stanol amount was added continuously in-line as a 30 wt-% solution in methanol before the feed entered the evaporator. Evaporator temperature was 125°C and pressure varied from 1.7 to 2.5 kPa. Methanol formed during the reaction was transferred as vapour from the evaporator and was condensed outside the vacuum system. Plant stanol ester was collected continuously as a residue from the evaporator bottom and the degree of esterification was 86.6%.

The same procedure was repeated but now a mixer unit was utilised for improving the mixing of catalyst added to the dry feed. Therefore, after having added the methanol containing the catalyst to the feed a static mixer (Vepu V-Mix L= 180 mm) was included in the device before the feed entered the evaporator. The residence time of the reaction mixture in between the catalyst addition and evaporator was 10 s, the temperature was 122 °C and the pressure 180 kPa. The evaporator temperature was 125°C and the pressure varied from 1.7 to 2.5 kPa. Methanol formed during the reaction was transferred as vapour from the evaporator and was condensed outside the vacuum system. Plant stanol ester was collected continuously as a residue from the evaporator bottom and the degree of esterification was 90.1%.

Serious splashing was observed in the evaporator during both runs causing unstable process conditions, such as vacuum fluctuation and some product loss to the vacuum system. This was due to the uneven feed distribution in the evaporator because of immediate evaporation of a high amount of methanol. The outcoming reaction product of plant stanol ester was foaming due to the unfinished reaction.

In order to find stable process conditions in the evaporator, the test was continued by reducing gradually the feed rate of the reaction mixture. Process design was retained as described in the second run and sodium methylate addition level was kept at 0.17 wt-% based on the stanol amount. Stable process conditions without splashing and foaming were found only at 12 kg/h feeding rate of the reaction mixture. The evaporator pressure was 1.7 kPa and temperature 125 °C. The obtained degree of esterification was 95.0 %.

### Example 3

### The use of a thin film evaporator as reactor; effect of recirculation

After noticing the problems described in Example 2 a new test with recirculation was performed by introducing a certain part of the reaction product that had already passed the short path evaporator to reaction mixture going into the short path evaporator.

50.0 kg of rapeseed oil methyl ester and 50.0 kg of plant stanols (molar ratio 1.4:1) were loaded into a feed vessel and this mixture was dried to remove water at 120°C and 1 kPa for 30 minutes with agitation. After drying the feed was pumped continuously from the feed tank to the reactor system consisting of a short path evaporator with a heating area of 0.45 m².

The ratio of recirculated reaction product to fresh reaction mixture was 3:1 (weight/weight) in the feed of the short path evaporator, and the feeding rate was approx. 61 kg/h. Catalyst dosing was 0.17 wt-% of sodium methylate catalyst based on the fresh stanol amount from the native feed in the reaction mixture. The catalyst was added continuously in-line as a 30 wt-% solution in methanol into the native dried feed and mixed by a static mixer (Vepu V-Mix L= 180 mm) before the reaction mixture entered the evaporator. The residence time of the reaction mixture (including both native reaction mixture and re-circulated reaction product) in between the catalyst addition and evaporator was 10 seconds, the temperature was 120°C and the line pressure 180 kPa. The evaporator pressure was 1.7 kPa and temperature 125°C. Plant stanol ester was collected continuously as a residue from the evaporator bottom at 15 kg/h flow rate and the rest was recirculated. The degree of esterification was calculated to 95.2%.

No foaming and splashing were observed in this recirculation processing mode and the reaction conditions were stable. However, to achieve enough high stanol ester formation, partly recycling through a thin film evaporator is required, which decreases the production capacity dramatically. Thus, the very high investment cost of a thin film evaporator will be poorly used.

### Example 4

### A pre-reactor unit in combination with thin film evaporator

50.0 kg of rapeseed oil methyl ester and 50.0 kg of plant stanols (molar ratio 1.4:1) were loaded into a feed vessel and this mixture was dried to remove water at 120°C and 1 kPa for 30 minutes with agitation. After drying the feed was pumped continuously from the feed tank with a feed rate of 61 kg/h to the reactor system consisting of a steam jacketed pre-reactor flash tank with a working volume of 18 dm³ (the height:diameter ratio was 1.64:1) including a feed distributor disk and a thin film evaporator (short path evaporator) with a heating area of 0.45 m². 0.17 wt-% of sodium methylate catalyst based on the stanol amount was added in-line as a 30 wt-% solution in methanol and mixed by a static mixer (Vepu V-Mix L= 180 mm) before the reaction mixture entered the pre-reactor unit. The residence time of the reaction mixture in between the catalyst addition and the pre-reactor was 10 s, the temperature was 122 °C and the line pressure 180 kPa. The evaporator temperature was 125°C and pressure 1.7 kPa. Methanol formed during the reaction was transferred as vapor from the reactor system and condensed outside the vacuum system. Plant stanol ester was collected continuously as a residue from the evaporator bottom and the degree of esterification was calculated to 95.4%.

Neither foaming nor splashing was observed and the reaction conditions were stable during the test.

The test was continued with the above described process design by introducing a certain part of the reaction product that had already passed the short path evaporator to reaction mixture going into the pre-reactor. The ratio of re-circulated reaction product to fresh reaction mixture was 1:3 (weight/weight) in the feed of the pre-reactor, and the feeding rate of the pre-reactor was approx. 61 kg/h. Sodium methylate dosing was 0.17 wt-% based on the fresh stanol amount from the native feed in the reaction mixture. The catalyst was added continuously in-line as a 30 wt-% solution in methanol into the native dried feed and mixed by a static mixer (Vepu V-Mix L= 180 mm) before the reaction mixture entered the pre-reactor unit. The residence time of the reaction mixture (including both native reaction mixture and recirculated reaction product) in between the catalyst addition and pre-reactor was 10 s, the temperature was 122°C and the line pressure 180 kPa. The pressure at the evaporator was 1.7 kPa and the temperature 125°C. Plant stanol ester was collected continuously as a residue from the evaporator bottom at approx. 45 kg/h flow rate and the rest was re-circulated. The degree of esterification was calculated to 98.1%.

Neither foaming nor splashing was observed and the reaction conditions were stable during the test.

## Claims

1. A process for the preparation of a sterol and/or stanol fatty acid ester by a continuous transesterification process, comprising providing a dried feed mixture comprising a sterol and/or stanol and an alkyl fatty acid ester, adding a transesterification catalyst to the dried feed mixture to obtain a reaction mixture, continuously feeding the reaction mixture into a thin film evaporator where the transesterification reaction takes place, continuously withdrawing alkanol formed during the reaction from the thin film evaporator, and continuously recovering a sterol and/or stanol fatty acid ester product from the thin film evaporator, and removing catalyst from the recovered product, and optionally refining the recovered product, wherein said reaction mixture entering the thin film evaporator comprises a partly transesterified reaction mixture.

2. The process according to claim 1, wherein the pressure of the reaction mixture in between the catalyst addition and the thin film evaporator is in the range from 10 to 1000 kPa, preferably from 50 to 500 kPa, the temperature is in the range from 100 to 170°C, preferably from 120 to 140 °C, and the residence time of the reaction mixture in between the catalyst addition and thin film evaporator is from 0.1 to 180 seconds, preferably from 0.2 to 60 seconds, and more preferably from 0.5 to 30 seconds.

3. The process according to claim 1, wherein the partly transesterified reaction mixture entering the thin film evaporator is obtained by continuously feeding the reaction mixture into a pre-reactor unit where the transesterification reaction is initiated and/or maintained and alkanol formed during the reaction is removed, wherein the pre-reactor unit comprises a vapour-liquid separator such as a flash tank, flash separator, degasser, cyclone separator, packed or tray column or a reactor equipped with an agitator, preferably a flash tank, flash separator, degasser, cyclone separator, packed or tray column, more preferably a flash tank, flash separator, degasser or cyclone separator.

4. The process according to claim 3, wherein the pre-reactor unit operates at an elevated temperature in the range of from 90 to 200°C, preferably from 100 to 170°C, and more preferably from 110 to 150°C, and at a low pressure of at most 95 kPa, preferably at most 10 kPa and more preferably at most 3 kPa, and the residence time of the reaction mixture in the pre-reactor unit is from 0.1 to 300 seconds, preferably from 0.2 to 180 seconds, and more preferably from 0.5 to 60 seconds.

5. The process according to claim 3 or 4, wherein the degree of esterification after the pre-reactor unit is from 5 to 94%, preferably from 10 to 90%, more preferably from 15 to 85%, even more preferably from 20 to 80%, and most preferably from 30 to 70%.

6. The process according to any one of claims 3 to 5, wherein the partly transesterified reaction mixture entering the thin film evaporator is obtained by combining a continuously recirculated part of the sterol and/or stanol fatty acid ester product recovered from the thin film evaporator with the reaction mixture before and/or after the pre-reactor unit, and/or by combining a continuously recirculated part of the sterol and/or stanol fatty acid ester product recovered from the thin film evaporator with the dried feed mixture and adding catalyst therein to form the partly transesterified reaction mixture before the pre-reactor unit, wherein the weight ratio of the recirculated part of the sterol and/or stanol fatty acid ester product to native reaction mixture is preferably from 0.01:1 to 10:1, more preferably from 0.05:1 to 5:1, still more preferably from 0.1:1 to 3:1 and most preferably from 0.1:1 to 0.5:1.

7. The process according to claim 1 or 2, wherein the partly transesterified reaction mixture entering the thin film evaporator is obtained by combining the reaction mixture with a continuously recirculated part of the sterol and/or stanol fatty acid ester product recovered from the thin film evaporator and/or by combining the dried feed mixture with a continuously recirculated part of the sterol and/or stanol fatty acid ester product recovered from the thin film evaporator and adding the catalyst, wherein the weight ratio of the recirculated part of the sterol and/or stanol fatty acid ester product to native reaction mixture is preferably from 0.1:1 to 10:1, more preferably from 1:1 to 5:1, most preferably from 1:1 to 3:1.

8. The process according to any one of claims 1 to 7, wherein the transesterification catalyst comprises an alkali metal or an alkali metal alcoholate, preferably sodium methylate or sodium ethylate, wherein the amount of the transesterification catalyst is preferably from 0.01 to 2% by weight, more preferably from 0.05 to 1% by weight and most preferably from 0.1 to 0.5% by weight of the sterol and/or stanol reactant.

9. The process according to any one of claims 1 to 8, wherein the degree of esterification after the thin film evaporator is at least 90%, preferably at least 95%, and more preferably at least 98%.

10. The process according to any one of claims 1 to 9, wherein the alkyl residue of the alkyl fatty acid ester is an alkyl group having from 1 to 10 carbon atoms, preferably methyl or ethyl.

11. The process according to any one of claims 1 to 10, wherein the fatty acid residue of the alkyl fatty acid ester is a carboxylic acid group having from 2 to 24 carbon atoms, preferably having from 12 to 20 carbon atoms.

12. The process according to any one of claims 1 to 11, wherein the fatty acid residue is obtained from a vegetable fat or oil or marine fat or oil.

13. A device for the continuous preparation of a sterol and/or stanol fatty acid ester by a continuous transesterification process, said device comprising
means (4) for providing a continuous stream of dried feed mixture comprising a sterol and/or stanol and an alkyl fatty acid ester;
means (3) for continuous introduction of a transesterification catalyst into the dried feed mixture to obtain a reaction mixture;
means for continuous feeding of the reaction mixture into a thin film evaporator (7), said thin film evaporator being equipped with inlet means for introduction of the reaction mixture, first outlet means for continuous withdrawal of a sterol and/or stanol fatty acid ester product from the thin film evaporator and second outlet means (8) for continuous removal of volatile components; and
means for regulation of temperature and pressure,
said device additionally comprising a pre-reactor unit (5) being positioned in the flow direction before the thin film evaporator (7), wherein the pre-reactor unit is a reaction vessel including means (6) for removal of vapours, and wherein the pre-reactor unit (5) contains no mechanical agitation and is a flash tank, flash separator, degasser, cyclone separator, packed or tray column.

14. The device according to claim 13, wherein the pre-reactor unit is a flash tank, flash separator, degasser or cyclone separator.

15. The device according to claim 14 additionally comprising means (12, 13, 14, 14') for continuous recirculation of part of the sterol and/or stanol fatty acid ester product withdrawn from the thin film evaporator (7) into any of the following: the reaction mixture entering the pre-reactor unit (5), the partly esterified reaction mixture entering the thin film evaporator (7) and the dried feed mixture, or any combination thereof.

16. The device according to any one of claims 13 to 15 additionally comprising a catalyst removal unit (9) and a refining unit (10), and optionally a unit for continuous drying of feed mixture to provide said continuous stream of dried feed mixture.

## Patentansprüche

1. Verfahren zur Herstellung eines Sterol- und/oder Stanolfettsäureesters durch ein kontinuierliches Umesterungsverfahren, umfassend Vorsehen einer trockenen Einspeisemischung, umfassend ein Sterol und/oder Stanol und einen Alkylfettsäureester, Zugeben eines Umesterungskatalysators zu der trockenen Einspeisemischung, um eine Reaktionsmischung zu erhalten, kontinuierliches Einspeisen der Reaktionsmischung in einen Dünnschichtverdampfer, wo die Umesterungsreaktion stattfindet, kontinuierliches Abziehen von während der Reaktion gebildetem Alkanol von dem Dünnschichtverdampfer, und kontinuierliches Gewinnen eines Sterol- und/der Stanolfettsäureesterprodukts aus dem Dünnschichtverdampfer, und Entfernen von Katalysator aus dem gewonnen Produkt, und wahlweise Raffmieren des gewonnen Produkts, wobei die in den Dünnschichtverdampfer eintretende Reaktionsmischung eine teilweise umgeesterte Reaktionsmischung umfasst.

2. Verfahren nach Anspruch 1, wobei der Druck der Reaktionsmischung zwischen der Katalysatorzugabe und dem Dünnschichtverdampfer im Bereich von 10 bis 1000 kPa, vorzugsweise von 50 bis 500 kPa liegt, die Temperatur in dem Bereich von 100 bis 170 °C, vorzugsweise von 120 bis 140 °C liegt, und die Verweilzeit der Reaktionsmischung zwischen der Katalysatorzugabe und dem Dünnschichtverdampfer 0,1 bis 180 Sekunden, vorzugsweise 0,2 bis 60 Sekunden, und weiter vorzugsweise 0,5 bis 30 Sekunden beträgt.

3. Verfahren nach Anspruch 1, wobei die teilweise umgeesterte Reaktionsmischung, welche in den Dünnschichtverdampfer eintritt, erhalten wird durch kontinuierliches Einspeisen der Reaktionsmischung in eine Vorreaktoreinheit, wo die Umesterungsreaktion initüert und/oder aufrechterhalten wird und während der Reaktion gebildetes Alkanol entfernt wird, wobei die Vorreaktoreinheit einen Dampf-Flüssigkeit-Separator, wie einen Entspannungstank, Entspannungsseparator, Entgaser, Zyklonseparator, Pack- oder Bodenkolonne oder einen mit einem Agitator ausgerüsteten Reaktor, vorzugsweise einen Entspannungstank, Entspannungsseparator, Entgaser, Zyklonseparator, Pack- oder Bodenkolonne, weiter vorzugsweise einen Entspannungstank, Entspannungsseparator, Entgaser oder Zyklonseparator, umfasst.

4. Verfahren nach Anspruch 3, wobei die Vorreaktoreinheit bei einer erhöhten Temperatur in dem Bereich von 90 bis 200 °C, vorzugsweise von 100 bis 170 °C und weiter vorzugsweise von 110 bis 150 °C, und bei einem niedrigen Druck von höchstens 95 kPa, vorzugsweise höchstens 10 kPa und weiter vorzugsweise höchstens 3 kPa, betrieben wird und die Verweilzeit der Reaktionsmischung in der Vorreaktoreinheit 0,1 bis 300 Sekunden, vorzugsweise 0,2 bis 180 Sekunden, und weiter vorzugsweise 0,5 bis 60 Sekunden beträgt.

5. Verfahren nach Anspruch 3 oder 4, wobei der Veresterungsgrad nach der Vorreaktoreinheit 5 bis 94 %, vorzugsweise 10 bis 90 %, weiter vorzugsweise 15 bis 85 %, noch weiter vorzugsweise 20 bis 80 %, und am meisten bevorzugt 30 bis 70 %, beträgt.

6. Verfahren nach irgendeinem der Ansprüche 3 bis 5, wobei die teilweise umgeesterte Reaktionsmischung, welche in den Dünnschichtverdampfer eintritt, erhalten wird durch Kombinieren eines kontinuierlich rezirkulierten Teils des von dem Dünnschichtverdampfer gewonnen Sterol- und/oder Stanolfettsäureesterprodukts mit der Reaktionsmischung vor und/oder nach der Vorreaktoreinheit, und/oder durch Kombinieren eines kontinuierlich rezirkulierten Teils des von dem Dünnschichtverdampfer gewonnen Sterol- und/oder Stanolfettsäureesterprodukts mit der trockenen Einspeisemischung und Zugeben von Katalysator hierzu zur Bildung der teilweise umgeesterten Reaktionsmischung vor der Vorreaktoreinheit, wobei das Gewichtsverhältnis des rezirkulierten Teils des Sterol- und/oder Stanolfettsäureesterprodukts zu der nativen Reaktionsmischung vorzugsweise 0,01:1 bis 10:1, weiter vorzugsweise 0,05:1 bis 5:1, noch weiter vorzugsweise 0,1:1 bis 3:1, und am meisten bevorzugt 0,1:1 bis 0,5:1, beträgt.

7. Verfahren nach Anspruch 1 oder 2, wobei die teilweise ungeesterte Reaktionsmischung, welche in den Dünnschichtverdampfer eintritt, erhalten wird durch Kombinieren der Reaktionsmischung mit einem kontinuierlich rezirkulierten Teil des von dem Dünnschichtverdampfer gewonnen Sterol- und/oder Stanolfettsäureesterprodukts und/oder durch Kombinieren der trockenen Einspeisemischung mit einem kontinuierlich rezierkulierten Teil des von dem Dünnschichtverdampfer gewonnen Sterol- und/oder Stanolfettsäureesterprodukts und Zugeben des Katalysators, wobei das Gewichtsverhältnis des rezirkulierten Teils des Sterol- und/oder Stanolfettsäureesterprodukts zu nativer Reaktionsmischung vorzugsweise 0,1:1 bis 10:1, weiter vorzugsweise 1:1 bis 5:1, am meisten bevorzugt 1:1 bis 3:1, beträgt.

8. Verfahren nach irgendeinem der Ansprüche 1 bis 7, wobei der Umesterungskatalysator ein Alkalimetall oder ein Alkalimetallalkoholat umfasst, vorzugsweise Natriummethylat oder Natriumethylat, wobei die Menge des Umsterungskatalysators vorzugsweise 0,01 bis 2 Gew.-%, weiter vorzugsweise 0,05 bis 1 Gew.-%, und am meisten bevorzugt 0,1 bis 0,5 Gew.-% des Sterol- und/oder Stanolreaktanten beträgt.

9. Verfahren nach irgendeinem der Ansprüche 1 bis 8, wobei der Veresterungsgrad nach dem Dünnschichtverdampfer mindestens 90 %, vorzugsweise mindestens 95 %, und am meisten bevorzugt mindestens 98 % beträgt.

10. Verfahren nach irgendeinem der Ansprüche 1 bis 9, wobei der Alkylrest des Alkylfettsäureesters eine Alkylgruppe mit 1 bis 10 Kohlenstoffatomen, vorzugsweise Methyl oder Ethyl, ist.

11. Verfahren nach irgendeinem der Ansprüche 1 bis 10, wobei der Fettsäurerest des Alkylfettsäureesters eine Carbonsäuregruppe mit 2 bis 24 Kohlenstoffatomen, vorzugsweise mit 12 bis 20 Kohlenstoffatomen, ist.

12. Verfahren nach irgendeinem der Ansprüche 1 bis 11, wobei der Fettsäurerest von einem pflanzlichen Fett oder Öl oder Marinefett oder -öl erhalten wird.

13. Vorrichtung zur kontinuierlichen Herstellung eines Sterol- und/oder Stanolfettsäureesters durch ein kontinuierliches Umesterungsverfahren, wobei die Vorrichtung umfasst
Mittel (4) zum Vorsehen eines kontinuierlichen Stroms einer trockenen Einspeisemischung, umfassend ein Sterol und/oder Stanol und einen Alkylfettsäureester;
Mittel (3) zur kontinuierlichen Einführung eines Umesterungskatalysators in die trockene Einspeisemischung, um eine Reaktionsmischung zu erhalten;
Mittel zum kontinuierlichen Einspeisen der Reaktionsmischung in einen Dünnschichtverdampfer (7), wobei der Dünnschichtverdampfer ausgerüstet ist mit Einlassmitteln zur Einführung der Reaktionsmischung, ersten Auslassmitteln zum kontinuierlichen Abziehen eines Sterol- und/oder Stanolfettsäureesterprodukts von dem Dünnschichtverdampfer, und zweiten Auslassmitteln (8) zur kontinuierlichen Entfernung von flüchtigen Komponenten; und
Mittel zur Regulierung von Temperatur und Druck,
wobei die Vorrichtung zusätzlich eine Vorreaktoreinheit (5) umfasst, welche in der Strömungsrichtung vor dem Dünnschichtverdampfer (7) positioniert ist, wobei die Vorreaktoreinheit ein Reaktionskessel ist, einschließlich Mitteln (6) zur Entfernung von Dämpfen, und wobei die Vorreaktoreinheit (5) keine mechanische Rührung umfasst und ein Entspannungstank, Entspannungsseparator, Entgaser, Zyklonseparator, Pack- oder Bondenkolonne ist.

14. Vorrichtung nach Anspruch 13, wobei die Vorreaktoreinheit ein Entspannungstank, Entspannungsseparator, Entgaser oder Zyklonseparator ist.

15. Vorrichtung nach Anspruch 14, zusätzlich umfassend Mittel (12, 13, 14, 14') zur kontinuierlichen Rezirkulation eines Teils des von dem Dünnschichtverdampfer (7) abgezogenen Sterol- und/oder Stanolfettsäureesterprodukts in irgendeines der Folgenden: der Reaktionsmischung, welche in die Vorreaktoreinheit (5) eintritt, die teilweise veresterte EReaktionsmischung, welche in den Dünnschichtverdampfer (7) eintritt, und die trockene Einspeisemischung oder irgendeine Kombination davon.

16. Vorrichtung nach irgendeinem der Ansprüche 13 bis 15, zusätzlich umfassend eine Katalysatorentfernungseinheit (9) und eine Raffmiereinheit (10), und wahlweise eine Einheit zur kontinuierlichen Trocknung von Einspeisemischung, um den kontinuierlichen Strom der trockenen Einspeisemischung vorzusehen.

## Revendications

1. Procédé de préparation d'un ester d'acide gras de stérol et/ou de stanol par un procédé de transestérification continue, comprenant la fourniture d'un mélange d'alimentation séché comprenant un stérol et/ou un stanol et un ester d'acide gras d'alkyle, l'ajout d'un catalyseur de transestérification au mélange d'alimentation séché pour obtenir un mélange réactionnel, l'alimentation continue du mélange réactionnel dans un évaporateur à couche mince où la réaction de transestérification a lieu, l'extraction continue de l'alcanol formé au cours de la réaction de l'évaporateur à couche mince, et la récupération continue d'un produit d'ester d'acide gras de stérol et/ou de stanol à partir de l'évaporateur à couche mince, et l'élimination du catalyseur du produit récupéré, et facultativement le raffinage du produit récupéré, dans lequel ledit mélange réactionnel entrant dans l'évaporateur à couche mince comprend un mélange réactionnel partiellement transestérifié.

2. Procédé selon la revendication 1, dans lequel la pression du mélange réactionnel entre l'ajout du catalyseur et l'évaporateur à couche mince se situe dans la plage de 10 à 1000 kPa, de préférence de 50 à 500 kPa, la température se situe dans la plage de 100 à 170 °C, de préférence de 120 à 140 °C, et le temps de séjour du mélange réactionnel entre l'ajout du catalyseur et l'évaporateur à couche mince est de 0,1 à 180 secondes, de préférence de 0,2 à 60 secondes, et plus préférablement de 0,5 à 30 secondes.

3. Procédé selon la revendication 1, dans lequel le mélange réactionnel partiellement transestérifié entrant dans l'évaporateur à couche mince est obtenu par alimentation continue du mélange réactionnel dans une unité de pré-réacteur où la réaction de transestérification est amorcée et/ou maintenue et l'alcanol formé au cours de la réaction est éliminé, dans lequel l'unité de pré-réacteur comprend un séparateur vapeur-liquide tel qu'un ballon de détente, un séparateur éclair, un dégazeur, un séparateur cyclone, une colonne à garnissage ou à plateaux ou un réacteur équipé d'un agitateur, de préférence un ballon de détente, un séparateur éclair, un dégazeur, un séparateur cyclone, une colonne à garnissage ou à plateaux, plus préférablement un ballon de détente, un séparateur éclair, un dégazeur ou un séparateur cyclone.

4. Procédé selon la revendication 3, dans lequel l'unité de pré-réacteur fonctionne à une température élevée dans la plage de 90 à 200 °C, de préférence de 100 à 170 °C, et plus préférablement de 110 à 150 °C, et à une pression basse d'au plus 95 kPa, de préférence d'au plus 10 kPa et plus préférablement d'au plus 3 kPa, et le temps de séjour du mélange réactionnel dans l'unité de pré-réacteur est de 0,1 à 300 secondes, de préférence de 0,2 à 180 secondes, et plus préférablement de 0,5 à 60 secondes.

5. Procédé selon la revendication 3 ou 4, dans lequel le degré d'estérification après l'unité de pré-réacteur est de 5 à 94 %, de préférence de 10 à 90 %, plus préférablement de 15 à 85 %, encore plus préférablement de 20 à 80 %, et de manière préférée entre toutes de 30 à 70 %.

6. Procédé selon l'une quelconque des revendications 3 à 5, dans lequel le mélange réactionnel partiellement transestérifié entrant dans l'évaporateur à couche mince est obtenu par combinaison d'une partie continuellement remise en circulation du produit d'ester d'acide gras de stérol et/ou de stanol récupéré à partir de l'évaporateur à couche mince avec le mélange réactionnel avant et/ou après l'unité de pré-réacteur, et/ou par combinaison d'une partie continuellement remise en circulation du produit d'ester d'acide gras de stérol et/ou de stanol récupéré à partir de l'évaporateur à couche mince avec le mélange d'alimentation séché et ajout du catalyseur dans celui-ci pour former le mélange réactionnel partiellement transestérifié avant l'unité de pré-réacteur, dans lequel le rapport pondéral de la partie remise en circulation du produit d'ester d'acide gras de stérol et/ou de stanol au mélange réactionnel initial est de préférence de 0,01/1 à 10/1, plus préférablement de 0,05/1 à 5/1, encore plus préférablement de 0,1/1 à 3/1 et de manière préférée entre toutes de 0,1/1 à 0,5/1.

7. Procédé selon la revendication 1 ou 2, dans lequel le mélange réactionnel partiellement transestérifié entrant dans l'évaporateur à couche mince est obtenu par combinaison du mélange réactionnel avec une partie continuellement remise en circulation du produit d'ester d'acide gras de stérol et/ou de stanol récupéré à partir de l'évaporateur à couche mince et/ou par combinaison du mélange d'alimentation séché avec une partie continuellement remise en circulation du produit d'ester d'acide gras de stérol et/ou de stanol récupéré à partir de l'évaporateur à couche mince et ajout du catalyseur, dans lequel le rapport pondéral de la partie remise en circulation du produit d'ester d'acide gras de stérol et/ou de stanol au mélange réactionnel initial est de préférence de 0,1/1 à 10/1, plus préférablement de 1/1 à 5/1, de manière préférée entre toutes de 1/1 à 3/1.

8. Procédé selon l'une quelconque des revendications 1 à 7, dans lequel le catalyseur de transestérification comprend un métal alcalin ou un alcoolat de métal alcalin, de préférence du méthylate de sodium ou de l'éthylate de sodium, dans lequel la quantité du catalyseur de transestérification est de préférence de 0,01 à 2 % en poids, plus préférablement de 0,05 à 1 % en poids et de manière préférée entre toutes de 0,1 à 0,5 % en poids du réactif de stérol et/ou de stanol.

9. Procédé selon l'une quelconque des revendications 1 à 8, dans lequel le degré d'estérification après l'évaporateur à couche mince est d'au moins 90 %, de préférence d'au moins 95 %, et plus préférablement d'au moins 98 %.

10. Procédé selon l'une quelconque des revendications 1 à 9, dans lequel le résidu alkyle de l'ester d'acide gras d'alkyle est un groupe alkyle comportant de 1 à 10 atomes de carbone, de préférence un groupe méthyle ou éthyle.

11. Procédé selon l'une quelconque des revendications 1 à 10, dans lequel le résidu acide gras de l'ester d'acide gras d'alkyle est un groupe acide carboxylique comportant de 2 à 24 atomes de carbone, de préférence comportant de 12 à 20 atomes de carbone.

12. Procédé selon l'une quelconque des revendications 1 à 11, dans lequel le résidu acide gras est obtenu à partir d'une graisse ou d'une huile végétale ou d'une graisse ou d'une huile de poisson.

13. Dispositif pour la préparation continue d'un ester d'acide gras de stérol et/ou de stanol par un procédé de transestérification continue, ledit dispositif comprenant
un moyen (4) de fourniture d'un courant continu de mélange d'alimentation séché comprenant un stérol et/ou stanol et un ester d'acide gras d'alkyle ;
un moyen (3) d'introduction continue d'un catalyseur de transestérification dans le mélange d'alimentation séché pour obtenir un mélange réactionnel ;
un moyen d'alimentation continue du mélange réactionnel dans un évaporateur à couche mince (7), ledit évaporateur à couche mince étant équipé d'un moyen d'entrée pour l'introduction du mélange réactionnel, un premier moyen de sortie pour l'extraction continue d'un produit d'ester d'acide gras de stérol et/ou de stanol de l'évaporateur à couche mince et un second moyen de sortie (8) pour l'élimination continue des composants volatils ; et
un moyen de régulation de la température et de la pression,
ledit dispositif comprenant en outre une unité de pré-réacteur (5) étant positionnée dans la direction de flux avant l'évaporateur à couche mince (7), dans lequel l'unité de pré-réacteur est une cuve de réaction incluant un moyen (6) d'élimination des vapeurs, et dans lequel l'unité de pré-réacteur (5) ne contient pas d'agitation mécanique et est un ballon de détente, un séparateur éclair, un dégazeur, un séparateur cyclone, une colonne à garnissage ou à plateaux.

14. Dispositif selon la revendication 13, dans lequel l'unité de pré-réacteur est un ballon de détente, un séparateur éclair, un dégazeur ou un séparateur cyclone.

15. Dispositif selon la revendication 14 comprenant en outre un moyen (12, 13, 14, 14') de remise en circulation continue de la partie du produit d'ester d'acide gras de stérol et/ou de stanol extraite de l'évaporateur à couche mince (7) dans l'un des suivants : le mélange réactionnel entrant dans l'unité de pré-réacteur (5), le mélange réactionnel partiellement estérifié entrant dans l'évaporateur à couche mince (7) et le mélange d'alimentation séché, ou toute combinaison de ceux-ci.

16. Dispositif selon l'une quelconque des revendications 13 à 15 comprenant en outre une unité d'élimination du catalyseur (9) et une unité de raffinage (10), et facultativement une unité pour le séchage continu du mélange d'alimentation pour fournir ledit courant continu de mélange d'alimentation séché.
